# EUROPÄISCHE PATENTANMELDUNG

(11) **EP 1 870 495 A1**
(43) Veröffentlichungstag der Anmeldung: **26.12.2007**
(21) Anmeldenummer: 06012766.9
(22) Anmeldetag: 21.06.2006
(51) Int. Cl.: C25D 3/22, C25D 3/56, C07D 211/00

(54) **Wässriges alkalisches cyanidfreies Bad zur galvanischen Abscheidung von Zink- und Zinklegierungsüberzügen**

(71) Anmelder: ATOTECH Deutschland GmbH, 10553 Berlin (DE)
(72) Erfinder: Brunner,Heiko, 10247 Berlin (DE); Kohlmann Lars, 12109 Berlijn (DE); Vogel Roland, 12247 Berlin (DE); Thom Konstatin, 13593 Berlin (DE)
(74) Vertreter: Albrecht, Thomas

(57) **Zusammenfassung**

Beschrieben wird ein wässriges alkalisches cyanidfreies Elektrolytbad zur Abscheidung von Zink- und Zinklegierungsschichten auf Substratoberflächen, das als Additive kationische heteroaromatische Stickstoffverbindungen als Glanzbildner enthält.

## Beschreibung

Die vorliegende Erfindung betrifft ein wässriges alkalisches galvanisches Bad ohne Zusatz von Cyanid-lonen als Komplexierungsreagenzien zur Abscheidung von Zink- und Zinklegierungsüberzügen, allen voran Zink-Nickel-, Zink-Eisen-und Zink-Cobalt-Überzügen, als Metallüberzug, die als Additive kationische heteroaromatische Stickstoffverbindungen enthalten, sowie ein Verfahren zur Abscheidung von glänzenden und ebenen Zink- und Zinklegierungsüberzügen, in dem das Bad verwendet wird. Ferner betrifft die Erfindung neue heteroaromatische Stickstoffverbindungen und ihre Verwendung als Glanzbildner.

Zinkabscheidungen aus cyanidischer, alkalischer Lösung dominieren seit vielen Jahren den industriellen Markt. Die immer höher werdenden gesetzlichen Auflagen hinsichtlich der Entsorgung alter Zink- und Zinkelektrolytbäder und die damit einhergehende strenge Kontrolle über das Abwasser führten zu einem verstärkten Interesse an nicht-cyanidischen Zink- und Zinklegierungsbädern. Dabei kommt gerade Zinklegierungelektrolyten eine besondere Bedeutung zu.

Derartige Metallüberzüge werden zur Verbesserung der Korrosionseigenschaften sowie zum Erreichen einer bestimmten Optik eingesetzt.

So wurde jahrzehntelang aufgalvanisiertes Zink von der Automobilindustrie verwendet, um einen wirtschaftlichen, hoch korrosionsbeständigen Überzug bereitzustellen. Mit den laufenden Forderungen nach höherer Qualität und umfassender Gewährleistung mussten jedoch sowohl die Automobilhersteller als auch die entsprechende Zulieferindustrie neue Überzugssysteme entwickeln. Dabei zeigen Zink-Nickel-Legierungsüberzüge hinsichtlich einer ausgedehnten Korrosionsbeständigkeit die besten Gesamtleistungen.

Es wurde gefunden, dass bei diesen Legierungsüberzügen die Nickelmenge in den galvanischen Zink-Nickel-Überzügen, welche für verbesserten Korrosionsschutz zweckdienlich ist, bei 4 bis 18% Nickel liegt, wobei besonders ein Anteil von 12 bis 15% optimal ist.

Im Stand der Technik sind sowohl saure als auch alkalische Zink-Nickel-Bäder bekannt. Die Schrift Modern Electroplating 4th edition (2000), S. 423-460 (Ed. M. Schlesinger und M. Paunovic) John Wiley & Sons gibt einen Überblick über technisch relevante galvanische Bäder.

Saure Zink-Nickel-Legierungsbäder basieren typischerweise auf anorganischen Zink- und Nickelsalzen, wie Zinksulfat, Zinkchlorid, Nickelsulfat oder Nickelchlorid, wobei diese verschiedene Additive enthalten, die sowohl den Glanz und die Kornstruktur verbessern als auch für eine Kontrolle des Zink/Nickel-Verhältnisses sorgen.

Saure Bäder für Zink-Nickel-Legierungsabscheidungen enthalten im allgemeinen eine Säure, wie z.B. Borsäure oder Schwefelsäure und andere Additive, wie z.B. Glanzbildner, Benetzungsmittel etc.

Nachteilig bei derartigen sauren Bädern ist die starke Korrosionswirkung des Elektrolyten auf die galvanische Anlage. Die alkalischen Bäder haben sich in der Praxis durchgesetzt, da sie eine bessere Metallverteilung besitzen und somit das Werkstück besser vor Korrosion schützen. Außerdem ist ihr Nickeleinbau über einen weiten Stromdichtebereich wesentlich gleichmäßiger, was mit einem besseren Korrosionsschutz einhergeht. Sie weisen jedoch eine geringere kathodische Stromausbeute auf, die zwischen 20 und 50% liegt. Ein solches Bad ist als Zink-Nickel-Bad in der Patentschrift US 3,681,211 beschrieben, die die Verwendung von Polyethylenimin lehrt, um einen schwarzen Überzug zu erreichen. Hierbei wird eine wässrige Zink-Nickel-Lösung mit Natronlauge auf einen pH-Wert zwischen 10 und 13 eingestellt. Durch die Verwendung von Polyethylenimin wird eine Komplexierung der Zink- und insbesondere der Nickel-Ionen erreicht, die beispielsweise als Zinkhydroxid- und Nickelhydroxid-Niederschlag in der Lösung vorliegen.

Die Verwendung von Polyethyleniminen hat seit Anfang der siebziger Jahre des zurückliegenden Jahrhunderts in Verbindung mit alkalischen Zink- und Zinklegierungsbädern eine weite Verwendung gefunden. Hierfür einsetzbare Polyethylenimine sind in der Patentschrift US 3,881,211 beschrieben und besitzen dort ein durchschnittliches Molekulargewicht von 600 bis 100000 und eine oft komplexe und verzweigte Struktur.

Die Schrift US 3,993,548 beschreibt ebenfalls die Verwendung eines hochmolekularen Polyethylenimins sowie quaternärer Ammonium-Silikate zur galvanischen Abscheidung glänzender, homogener Zinkschichten. Hierbei kann das Bad zusätzliche Glanzbildner, wie etwa Benzyl-betain-nicotinat enthalten.

Neben Polyethylenimen sind auch andere Komplexbildner als Komplexierungsreagenzien bekannt. So beschreibt die Schrift US 4,861,442 wässrige alkalische Bäder, die Zink- und Nickel-lonen, Alkalimetallhydroxid, ein Aminoalkohol-Polymer, ein Komplexierungsmittel für Nickel und eine Aminosäure und/oder ein Salz einer Aminosäure umfassen. Der pH-Wert des Bades ist 11 und höher.

Die Schrift US 4,877,496 beschreibt wässrige alkalische Bäder, die Zink- und Nickel-lonen, ein Alkalimetallhydroxid, ein Metallkomplexierungsmittel, einen primären Glanzbildner und einen Glanzverstärker umfassen. Der primäre Glanzbildner ist hierbei ein Kondensationsprodukt eines Amins, wie Ethylendiamin mit Epihalodrin. Der Glanzverstärker ist mindestens ein aromatischer Aldehyd. Tertiäre Glanzbildner, wie Telluroxid, tellurige Säure oder Tellursäure oder deren Salze können ebenfalls in den Bädern enthalten sein.

Die US 4,889,602 beschreibt wässrige Elektrolytbäder, die einen pH-Wert von mehr als 11 haben und Zink- und Nickel-lonen sowie mindestens eine Verbindung aus der Reihe aliphatische Amine, polymere aliphatische Amine und hydroxyaliphatische Carbonsäuren und deren Salze umfassen.

Die Schrift US 5,417,840 beschreibt die Verwendung eines Badsystems, das vor allem Polyalkylenimine als Komplexierungsreagenzien und Pyridinium-Betaine, allen voran Sulfobetaine enthält. Die Schrift DE 198 48 467 C2 beschreibt die Verwendung von Triethylentetramin, Tetraethylenpentamin oder Pentaethylentetramin als Komplexierungssystem in Kombination mit N-Benzylnicotinat-betain als primären Glanzbildner.

Benzylpyridinium-Verbindungen sind seit längerem als Glanzbildner für die Abscheidung von Zink-Schichten bekannt. So beschreibt die Schrift Kinzoku Hyomen Gijutsu (1980), 31, 244-248 den Einfluss 3-substituierter Pyridinium-Verbindungen auf die Textur der galvanisch abgeschiedenen Zink-Schichten. Die Patentschrift US 6,652,728 beschreibt die Verwendung von N-Benzylpyridinium-3-carboxylat und N,N'-p-Xylylen-bis-(pyridinium-3-carboxylat) als Glanzbildner in Kombination mit kationischen polymeren Harnstoff-Derivaten für alkalische Zink und Zinklegierungsbäder, vor allem Zink-Eisen-Bädern.

Bei allen in der Literatur bekannten Bädern zur Metallabscheidung besteht das Problem, dass häufig nicht über den gesamten Stromdichtebereich eine gleichmäßige Schicht bzw. eine gleichmäßige Glanzbildung erreicht wird. Oftmals werden auch Zink-Nickel-Schichten erhalten, die einen Nickelgehalt größer als 15% enthalten. Ferner zeigen die im Markt erhältlichen galvanischen Bäder zur Abscheidung von Zink-Nickel-Legierungsschichten oftmals nur eine mäßige kathodische Stromausbeute einhergehend mit einer starken Wasserstoffentwicklung.

Der Erfindung liegt deshalb die Aufgabe zugrunde, ein galvanisches Bad für Zink- und Zink-Legierungsabscheidungen zu entwickeln, welches optisch ansprechende Zink- und Zinklegierungs-Schichten ermöglicht. Bei der Abscheidung von Zinklegierungen soll zusätzlich eine homogenere ZinkLegierungsmetall-Verteilung und ein optimales Zink/Legierungsmetall-Verhältnis eingestellt werden. Dabei soll eine einheitliche Schichtdicke mit hohem Glanz und der Gleichmäßigkeit der Legierungskomponenten im Überzug über einen weiten Stromdichtebereich aufrechterhalten werden.

Gegenstand der Erfindung ist ein wässriges alkalisches, cyanid-freies Elektrolytbad zur Abscheidung von Zink- und Zinklegierungsschichten auf Substratoberflächen, umfassend die folgenden Komponenten:
a) eine Zink-Ionen-Quelle und gegebenenfalls eine Quelle für weitere Metallionen,
b) Hydroxidionen und
c) mindestens eine Pyridiniumverbindung der allgemeinen Formeln I bis III: 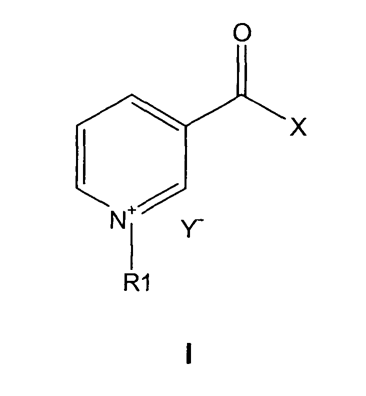 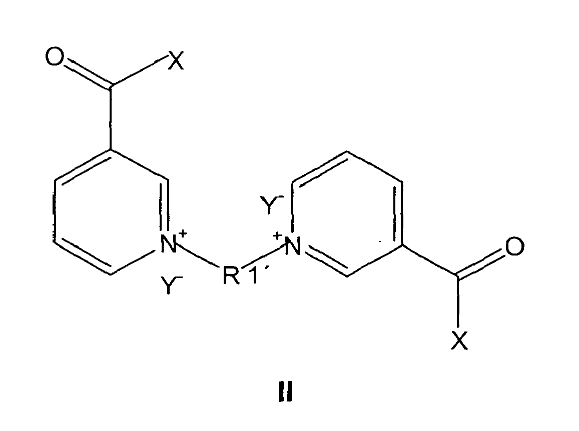 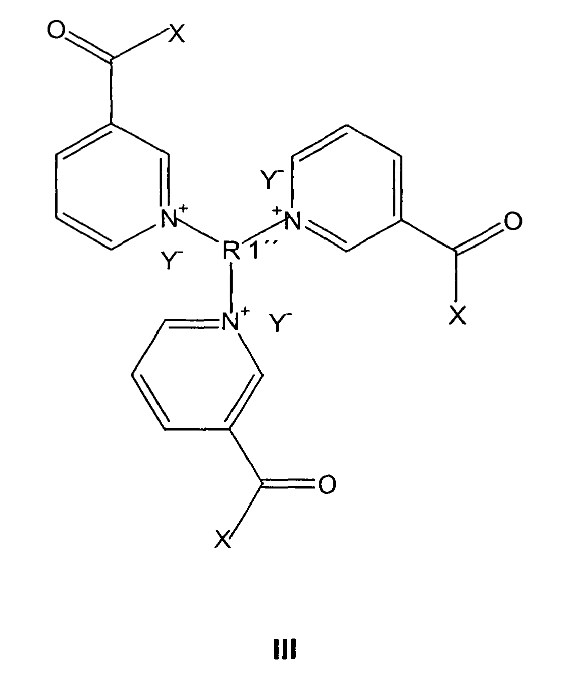 worin R¹ für einen substituierten oder unsubstituierten, gesättigten oder ungesättigten, aliphatischen oder araliphatischen Kohlenwasserstoffrest mit 1 bis 12 Kohlenstoffatomen steht,
   R¹' für einen zweiwertigen substituierten oder unsubstituierten, gesättigten oder ungesättigten, aliphatischen oder araliphatischen Kohlenwasserstoffrest mit 1 bis 12 Kohlenstoffatomen steht,
   R¹" für einen dreiwertigen substituierten oder unsubstituierten, gesättigten oder ungesättigten, aliphatischen oder araliphatischen Kohlenwasserstoffrest mit 1 bis 12 Kohlenstoffatomen steht,
   X für Hydroxyl oder NRₓR_{y} steht, worin Rₓ und R_{y} gleich oder verschieden sein können und für Wasserstoff oder lineare und/oder verzweigte Alkylgruppen mit 1 bis 12 Kohlenstoffatomen stehen und Y⁻ ein Gegenion ist.

Gemäß einer bevorzugten Ausführungsform betrifft die Erfindung ein wie oben definiertes Elektrolytbad, worin R¹ für substituiertes Aryl der nachfolgenden Formeln R1 a bis R1l steht: 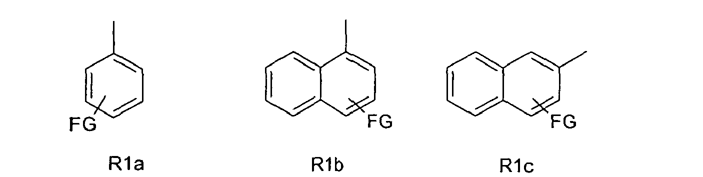 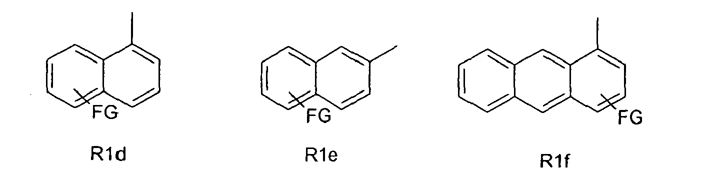 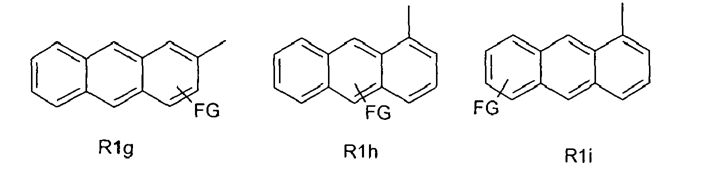 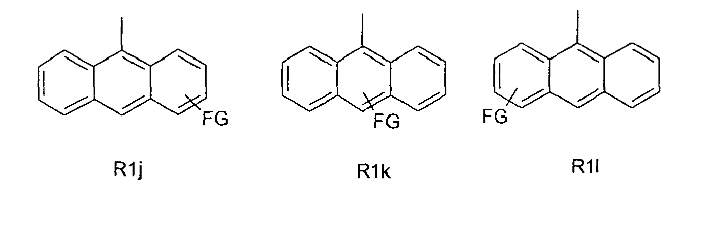 worin FG für einen Rest, ausgewählt aus der Gruppe, bestehend aus Carboxy-, Ester-, Sulfonsäure-, Carbamoyl-, Amino-, Cyano-, Alkyl-, Alkoxy-, Hydroxy-, Trifluormethyl-, Allyl-, Propargyl-, 4-Sulfobutyl-, 3-Sulfopropyl-, 4-Carboxybutyl-, 3-Carboxypropylresten, Wasserstoff und Halogenen, ausgewählt aus Fluor, Chlor und Brom, steht,
R1' für But-2-enyl, But-2-inyl oder für Aryl der nachfolgenden Formeln R1'a bis r steht: 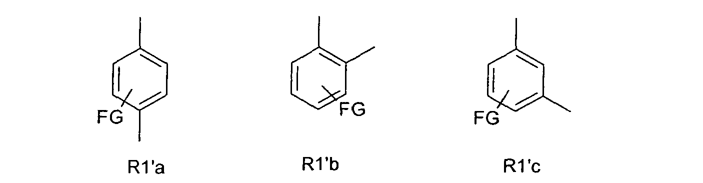 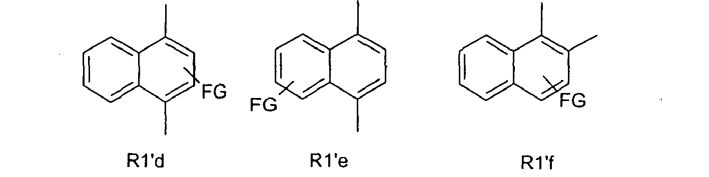 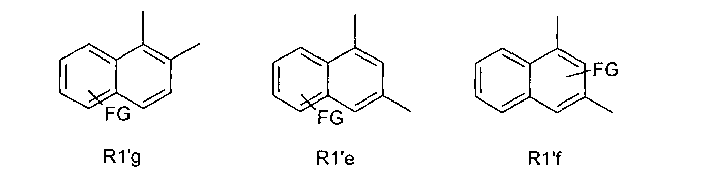 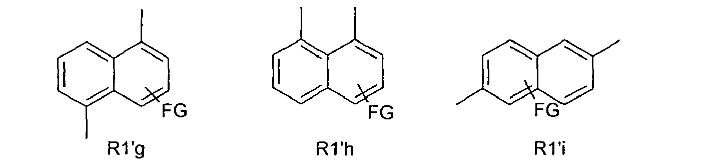 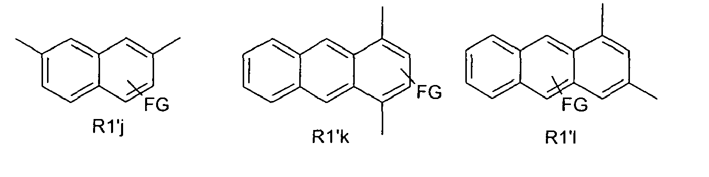 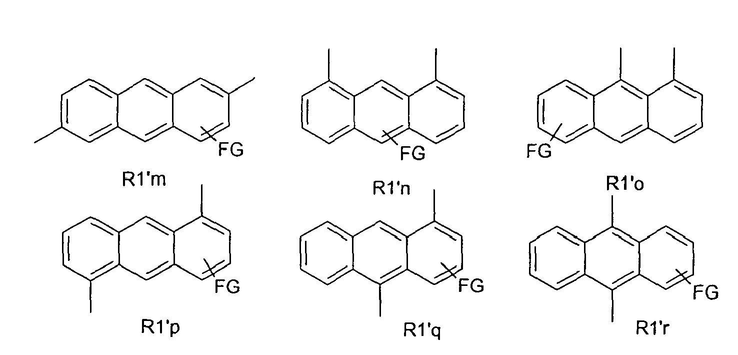 worin FG für einen Rest, ausgewählt aus der Gruppe, bestehend aus Carboxy-, Ester-, Sulfonsäure-, Carbamoyl-, Amino-, Cyano-, Alkyl-, Alkoxy-, Trifluormethylresten, Wasserstoff und Halogenen, ausgewählt aus Fluor, Chlor und Brom, steht, wobei alle Ringe oder einzelne anellierte Ringe substituiert sein können,
R¹" für 1,3,5-Mesityl, 1,2,4-Mesityl oder 1,2,3-Mesityl steht,
X für NH₂ oder OH steht und
Y ein Halogenid oder Pseudohalogenid ist.

Vorzugsweise sind die Reste R¹, R^{1'} und R^{1"} in den Formeln I bis III über eine Methylengruppe an den Pyridiniumrest gebunden.

Im Rahmen der vorliegenden Erfindung können als Halogenide Fluoride, Chloride und Bromide verwendet werden. Weiterhin können in dem erfindungsgemäßen Bad solche Verbindungen verwendet werden, die den Halogeniden hinsichtlich ihrer physikalischen und chemischen Eigenschaften ähnlich sind, d.h. so genannte Pseudohalogenide. Solche Pseudohalogenide sind dem Fachmann an sich bekannt und beispielsweise in dem Römpp-Lexikon, Chemie, 10. Auflage, Seite 3609 beschrieben. Im Rahmen der vorliegenden Erfindung umfassen die Pseudohalogenide auch Reste wie Mesitylat, Triflat etc.

Gemäß einer weiteren bevorzugten Ausführungsform der Erfindung enthält das Elektrolytbad weiterhin mindestens ein Komplexierungsreagenz der allgemeinen Formel IV oder V: 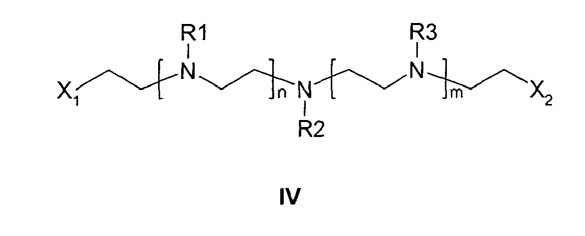 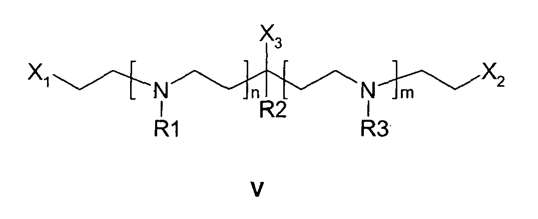 worin X₁, X₂ und X₃ gleich oder verschieden sein können und für eine Hydroxyl-, Alkoxy-, primäre, sekundäre oder tertiäre Aminogruppe stehen,
R¹, R² und R³ gleich oder verschieden sein können und für eine C₁-C₁₂-Alkoxyl-, -Allyl- oder -Propargylgruppe stehen und
n und m gleich oder verschieden sein können und für eine ganze Zahl von 0 bis 5 stehen.

Vorzugsweise sind weitere Komponenten in dem galvanischen Bad enthalten, um die Eigenschaften der abgeschiedenen Legierung zu verbessern. Dies können beispielsweise Polymere aliphatischer Amine und Metallkomplexierungsmittel sein.

Die erfindungsgemäßen Bäder umfassen eine wässrige alkalische Lösung, die Zink-Ionen, gegebenenfalls Ionen des Legierungsmetalls und mindestens eine der oben genannten aromatischen heterocyclischen stickstoffhaltigen Verbindungen enthält, wie das nachstehend genauer beschrieben wird.

Die erfindungsgemäßen galvanischen Bäder enthalten eine anorganische alkalische Komponente, vorzugsweise ein Hydroxid der Alkalimetalle und besonders bevorzugt Natriumhydroxid, um in dem Bad einen pH-Wert von mindestens 10 und vorzugsweise von mindestens 11 einzustellen. Hierbei können Mengen von 50 bis etwas 250 Gramm/Liter und besonders bevorzugt von 90 bis ca. 130 Gramm/Liter der alkalischen Komponente eingesetzt werden.

Die erfindungsgemäßen Elektrolytbäder enthalten gewöhnlich Zink-Ionen in Konzentrationen, die von etwa 1 bis etwa 100 g/l reichen, wobei Konzentrationen von 4 bis 30 g/l bevorzugt sind. Das Zink-Ion kann in dem erfindungsgemäßen Bad in Form eines löslichen Salzes, etwa Zinkoxid, Zinksulfat, Zinkcarbonat, Zinkacetat, Zinksulfamat, Zinkhydroxid, Zinktartrat etc. vorliegen.

Als Legierungsmetall enthalten die erfindungsgemäßen Bäder etwa 0,1 bis 50 g/l Metallionen. Geeignete Metallsalze sind Hydroxide, Sulfate, Carbonate, Ammoniumsulfate, Sulfamate, Acetate, Formiate und Halogenide, bevorzugt Chlorid und Bromid.

Bevorzugt enthalten die erfindungsgemäßen Bäder als Legierungsmetall etwa 0,1 bis 50 g/l Nickel-Ionen. Geeignete Nickelsalze sind Nickelhydroxid, Nickelsulfat, Nickelcarbonat, Ammonium-Nickelsulfat, Nickelsulfamat, Nickelacetat, Nickelformiat und Nickelhalogenide.

Die Zink- und Legierungsmetallquellen, die in den erfindungsgemäßen Elektrolytbädern verwendet werden können, können eine oder mehrere der vorstehend beschriebenen Nickel- und Zinkquellen umfassen.

Die erfindungsgemäßen Elektrolytbäder enthalten die oben genannten aromatischen heterocyclischen stickstoffhaltigen Verbindungen der allgemeinen Formeln I bis III zur nachhaltigen Verbesserung der Einebnung und Glanzbildung der abgeschiedenen Schichten über einen weiten Stromdichtebereich.

Bevorzugte Verbindungen der Formeln I bis III sind 1-Benzyl-3-carbamoyl-pyridinium-chlorid, 1-(2'-Chloro-benzyl)-3-carbamoyl-pyridinium-chlorid, 1-(2'-Fluoro-benzyl)-3-carbamoyl-pyridinium-chlorid, 1-(2'-Methoxy-benzyl)-3-carbamoyl-pyridinium-chlorid, 1-(2'-Carboxy-benzyl)-3-carbamoyl-pyridinium-chlorid, 1-(2'-Carbamoyl-benzyl)-3-carbamoyl-pyridinium-chlorid, 1-(3'-Chloro-benzyl)-3-carbamoyl-pyridinium-chlorid, 1-(3'-Fluoro-benzyl)-3-carbamoyl-pyridinium-chlorid, 1-(3'-Methoxy-benzyl)-3-carbamoyl-pyridinium-chlorid, 1-(3'-Carboxy-benzyl)-3-carbamoyl-pyridinium-chlorid, 1-(3'-Carbamoyl-benzyl)-3-carbamoyl-pyridinium-chlorid, 1-(4'-Chloro-benzyl)-3-carbamoyl-pyridinium-chlorid, 1-(4'-Fluoro-benzyl)-3-carbamoyl-pyridinium-chlorid, 1-(4'-Methoxybenzyl)-3-carbamoyl-pyridinium-chlorid, 1-(4'-Carboxy-benzyl)-3-carbamoyl-pyridinium-chlorid, 1-(4'-Carbamoyl-benzyl)-3-carbamoyl-pyridinium-chlorid, 1-Benzyl-3-carboxy-pyridinium-chlorid, 1-(2'-Chloro-benzyl)-3-carboxy-pyridinium-chlorid, 1-(2'-Fluoro-benzyl)-3-carboxy-pyridinium-chlorid, 1-(2'-Methoxybenzyl)-3-carboxy-pyridinium-chlorid, 1-(2'-Carboxy-benzyl)-3-carboxy-pyridinium-chlorid, 1-(2'-Carbamoyl-benzyl)-3-carboxy-pyridinium-chlorid, 1-(3'-Chloro-benzyl)-3-carboxy-pyridinium-chlorid, 1-(3'-Fluoro-benzyl)-3-carboxy-pyridinium-chlorid, 1-(3'-Methoxy-benzyl)-3-carboxy-pyridinium-chlorid, 1-(3'-Carboxy-benzyl)-3-carboxy-pyridinium-chlorid, 1-(3'-Carbamoyl-benzyl)-3-carboxy-pyridinium-chlorid, 1-(4'-Chloro-benzyl)-3-carboxy-pyridinium-chlorid, 1-(4'-Fluoro-benzyl)-3-carboxy-pyridinium-chlorid, 1-(4'-Methoxy-benzyl)-3-carboxy-pyridinium-chlorid, 1-(4'-Carboxy-benzyl)-3-carboxy-pyridinium-chlorid, 1-(4'-Carbamoyl-benzyl)-3-carboxy-pyridinium-chlorid, 1-Benzyl-3-carbamoyl-pyridinium-bromid, 1-(2'-Chloro-benzyl)-3-carbamoyl-pyridinium-bromid, 1-(2'-Fluoro-benzyl)-3-carbamoyl-pyridinium-bromid, 1-(2'-Methoxy-benzyl)-3-carbamoyl-pyridinium-bromid, 1-(2'-Carboxy-benzyl)-3-carbamoyl-pyridinium-bromid, 1-(2'-Carbamoyl-benzyl)-3-carbamoyl-pyridinium-bromid, 1-(3'-Chloro-benzyl)-3-carbamoyl-pyridinium-bromid, 1-(3'-Fluoro-benzyl)-3-carbamoyl-pyridinium-bromid, 1-(3'-Methoxy-benzyl)-3-carbamoyl-pyridinium-bromid, 1-(3'-Carboxy-benzyl)-3-carbamoyl-pyridinium-bromid, 1-(3'-Carbamoyl-benzyl)-3-carbamoyl-pyridinium-bromid, 1-(4'-Chloro-benzyl)-3-carbamoyl-pyridinium-bromid, 1-(4'-Fluoro-benzyl)-3-carbamoyl-pyridinium-bromid, 1-(4'-Methoxybenzyl)-3-carbamoyl-pyridinium-bromid, 1-(4'-Carboxy-benzyl)-3-carbamoyl-pyridinium-bromid, 1-(4'-Carbamoyl-benzyl)-3-carbamoyl-pyridinium-bromid, 1-Benzyl-3-carboxy-pyridinium-bromid, 1-(2'-Chloro-benzyl)-3-carboxy-pyridinium-bromid, 1-(2'-Fluoro-benzyl)-3-carboxy-pyridinium-bromid, 1-(2'-Methoxybenzyl)-3-carboxy-pyridinium-bromid, 1-(2'-Carboxy-benzyl)-3-carboxy-pyridinium-bromid, 1-(2'-Carbamoyl-benzyl)-3-carboxy-pyridinium-bromid, 1-(3'-Chloro-benzyl)-3-carboxy-pyridinium-bromid, 1-(3'-Fluoro-benzyl)-3-carboxy-pyridinium-bromid, 1-(3'-Methoxy-benzyl)-3-carboxy-pyridinium-bromid, 1-(3'-Carboxy-benzyl)-3-carboxy-pyridinium-bromid, 1-(3'-Carbamoyl-benzyl)-3-carboxy-pyridinium-bromid, 1-(4'-Chloro-benzyl)-3-carboxy-pyridinium-bromid, 1-(4'-Fluoro-benzyl)-3-carboxy-pyridinium-bromid, 1-(4'-Methoxy-benzyl)-3-carboxy-pyridinium-bromid, 1-(4'-Carboxy-benzyl)-3-carboxy-pyridinium-bromid, 1-(4'-Carbamoyl-benzyl)-3-carboxy-pyridinium-bromid,1-Benzyl-3-carbamoyl-pyridinium-fluorid, 1-(2'-Chloro-benzyl)-3-carbamoyl-pyridinium-fluorid, 1-(2'-Fluoro-benzyl)-3-carbamoyl-pyridinium-fluorid, 1-(2'-Methoxy-benzyl)-3-carbamoyl-pyridinium-fluorid, 1-(2'-Carboxy-benzyl)-3-carbamoyl-pyridinium-fluorid, 1-(2'-Carbamoyl-benzyl)-3-carbamoyl-pyridinium-fluorid, 1-(2'-Sulfo-benzyl)-3-carbamoyl-pyridinium-fluorid, 1-(3'-Chloro-benzyl)-3-carbamoyl-pyridinium-fluorid, 1-(3'-Fluoro-benzyl)-3-carbamoyl-pyridinium-fluorid, 1-(3'-Methoxy-benzyl)-3-carbamoyl-pyridinium-fluorid, 1-(3'-Carboxy-benzyl)-3-carbamoyl-pyridinium-fluorid, 1-(3'-Carbamoyl-benzyl)-3-carbamoyl-pyridinium-fluorid, 1-(4'-Chloro-benzyl)-3-carbamoyl-pyridinium-fluorid, 1-(4'-Fluoro-benzyl)-3-carbamoyl-pyridinium-fluorid, 1-(4'-Methoxy-benzyl)-3-carbamoyl-pyridinium-fluorid, 1-(4'-Carboxy-benzyl)-3-carbamoyl-pyridinium-fluorid, 1-(4'-Carbamoyl-benzyl)-3-carbamoyl-pyridinium-fluorid, 1-Benzyl-3-carboxy-pyridinium-fluorid, 1-(2'-Chloro-benzyl)-3-carboxy-pyridinium-fluorid, 1-(2'-Fluoro-benzyl)-3-carboxy-pyridinium-fluorid, 1-(2'-Methoxy-benzyl)-3-carboxy-pyridinium-fluorid, 1-(2'-Carboxy-benzyl)-3-carboxy-pyridinium-fluorid, 1-(2'-Carbamoyl-benzyl)-3-carboxy-pyridinium-fluorid, 1-(3'-Chloro-benzyl)-3-carboxy-pyridinium-fluorid, 1-(3'-Fluoro-benzyl)-3-carboxy-pyridinium-fluorid, 1-(3'-Methoxy-benzyl)-3-carboxy-pyridinium-fluorid,1-(3'-Carboxy-benzyl)-3-carboxy-pyridinium-fluorid, 1-(3'-Carbamoyl-benzyl)-3-carboxy-pyridinium-fluorid, 1-(4'-Chloro-benzyl)-3-carboxy-pyridinium-fluorid, 1-(4'-Fluoro-benzyl)-3-carboxy-pyridinium-fluorid, 1-(4'-Methoxy-benzyl)-3-carboxy-pyridinium-fluorid, 1-(4'-Carboxy-benzyl)-3-carboxy-pyridinium-fluorid, 1-(4'-Carbamoyl-benzyl)-3-carboxy-pyridinium-fluorid, 1-(1'-Methyl-naphthyl)-3-carboxy-pyridinium-chlorid, 1-(1'-Methyl-naphthyl)-3-carboxy-pyridinium-bromid, 1-(1'-Methyl-naphthyl)-3-carboxy-pyridinium-fluorid, 1 -(1'-Methyl-naphthyl)-3-carbamoyl-pyridinium-chlorid, 1-(1'methyl-naphthyl)-3-carbamoyl-pyridinium-bromid, 1-(1'-Methyl-naphthyl)-3-carbamoyl-pyridinium-fluorid, 1,1 '-(But-2-eny)-3,3'-bis-carbamoyl-bispyridinium-dichlorid, 1,1'-(But-2-enyl)-3,3'-bis-carbamoyl-bispyridinium-dibromid, 1,1'-(But-2-enyl)-3,3'-bis-carbamoyl-bispyridinium-difluorid, 1,1'-(But-2-enyl)-3,3'-bis-carboxy-bispyridinium-dichlorid, 1,1 '-(But-2-enyl)-3,3'-bis-carboxy-bispyridinium-dibromid, 1,1 '-(But-2-eny)-3,3'-bis-carboxy-bispyridinium-difluorid, 1-Allyl-3-carbamoyl-pyridinium-chlorid, 1-Allyl-3-carbamoyl-pyridinium-bromid, 1-Allyl-3-carbamoyl-pyridinium-fluorid, 1-Allyl-3-carboxy-pyridinium-chlorid, 1-Allyl-3-carboxy-pyridinium-bromid, 1-Allyl-3-carboxy-pyridinium-fluorid, 1-Propargyl-3-carbamoyl-pyridinium-chlorid, 1-Propargyl-3-carbamoyl-pyridinium-bromid, 1-Propargyl-3-carbamoyl-pyridinium-fluorid, 1-Propargyl-3-carboxy-pyridinium-chlorid, 1-Propargyl-3-carboxy-pyridinium-bromid, 1-Propargyl-3-carboxy-pyridinium-fluorid, 1,1'-(But-2-inyl)-3,3'-bis-carbamoyl-bispyridinium-dichlorid, 1,1'-(But-2-inyl)-3,3'-bis-carbamoyl-bispyridinium-dibromid, 1, 1'-(But-2-inyl)-3,3'-bis-carbamoyl-bispyridinium-difluorid, 1,1'-(But-2-inyl)-3,3'-bis-carboxy-bispyridinium-dichlorid, 1,1'-(But-2-inyl)-3,3'-bis-carboxy-bispyridinium-dibromid, 1,1'-(But-2-inyl)-3,3'-bis-carboxy-bispyridinium-difluorid, 1,1'-(Xylenyl)-3,3'-bis-carbamoyl-bis-pyridinium-dibromid, 1, 1 '-(Xylenyl)-3,3'-bis-carbamoyl-bis-pyridinium-difluorid, 1,1',1"-(Mesitylenyl)-3,3',3"-tris-carbamoyl-tri-pyridinium-trichlorid, 1,1',1"-(Mesitylenyl)-3,3',3"-tri-carboxy-tri-pyridinium-trichlorid, 1,1',1"-(Mesitylenyl)-3,3',3"-tris-carbamoyl-tri-pyridinium-tribromid, 1,1',1 "-(Mesitylenyl)-3,3',3"-tri-carboxy-tri-pyridinium-tribromid, 1,1',1"-(Mesitylenyl)-3,3',3"-tris-carbamoyl-tri-pyridinium-trifluorid, 1,1',1"-(Mesitylenyl)-3,3',3"-tri-carboxy-tri-pyridinium-trifluorid, 1-(3'-sulfopropyl)-3-carbamoyl-pyridinium-Betain sowie die korrespondierenden lodide und Pseudohalogenide (z.B. Triflate, Tosylate) der oben aufgeführten Verbindungen.

Die Glanzbildner können leicht durch Umsetzung der oben genannten Heterocyclen mit den entsprechenden Benzylhalogeniden in einem geeigneten Lösungsmittel, wie z.B. Ethanol, Propanol, iso-Propanol, Butanol, iso-Butanol, Methanol bzw. deren Gemischen oder auch in wässrigem Milieu in der Hitze dargestellt werden. Die hierzu benötigten Reaktionszeiten liegen je nach eingesetztem Einsatzstoff zwischen einer und 48 Stunden. Hierzu kann neben den klassischen Wärmequellen auch ein Mikrowellenofen eingesetzt werden. Die anfallenden Pyridiniumverbindungen können entweder als wässrige oder alkoholische Reaktionslösung direkt eingesetzt werden oder nach Abkühlen durch Filtration oder Entfernen des entsprechenden Lösemittels isoliert werden. Eine Aufreinigung der Verbindungen kann durch Umkristallisation in einem geeigneten Lösemittel wie z.B. Ethanol erfolgen.

Die Bispyridinium-Verbindungen der allgemeinen Formel IV können analog der Schrift US 6,652,728 hergestellt werden.

Dabei können oben genannte Verbindungen alleine oder als Gemisch der oben genannten erfindungsgemäßen Glanzbildner in einer Größenordnung von 0,001 bis 20 g/l und besonders bevorzugt von 0,01 bis 10 g/l eingesetzt werden.

Die erfindungsgemäßen Bäder für Zink-Nickel-Abscheidungen enthalten als Komplexierungsreagenzien Verbindungen der allgemeinen Formeln IV und V.

Die in den erfindungsgemäßen Bädern eingesetzten Mengen an Polyamin-Verbindungen liegen dabei in Abhängigkeit von der Zink- und Nickel-lonen-Konzentration zwischen 5 bis 100 g/l.

Beispiele geeigneter Komplexierungsreagenzien für die erfindungsgemäßen Bäder sind z.B. Diethylentriamin, Tetraethylenpentamin, Pentaethylenhexamin, 1,3-Bis[(2-hydroxy-ethyl)-methyl-amino]-propan-2-ol. Ferner können Komplexierungsreagenzien eingesetzt werden, wie sie in Schrift US 5,417,840 beschrieben sind.

Die erfindungsgemäßen Bäder können mit gebräuchlichen Verfahren hergestellt werden, beispielsweise durch Zugabe der spezifischen Mengen der vorstehend beschriebenen Komponenten zu Wasser. Die Menge der Basenkomponente, wie z.B. Natriumhydroxid, sollte ausreichen, um in dem Bad den gewünschten pH-Wert von mindestens 10 und vorzugsweise über 11 zu erreichen.

Die erfindungsgemäßen Bäder scheiden eine blanke, ebene und duktile Zink-oder Zinklegierungs-Schicht bei jeder gebräuchlichen Temperatur von etwa 15°C bis 50°C, vorzugsweise 20°C bis 30°C, besonders bevorzugt etwa 25°C. Bei diesen Temperaturen sind die erfindungsgemäßen Bäder stabil und über einen weiten Stromdichtebereich von 0,01 bis 10 A/dm² , besonders bevorzugt von 0,5 bis 4 A/dm² wirkungsvoll.

Die erfindungsgemäßen Bäder können auf eine kontinuierliche oder intermittierende Weise betrieben werden, und von Zeit zu Zeit wird man die Komponenten des Bades ergänzen müssen. Die Komponenten des Bades können einzeln oder in Kombination zugesetzt werden. Ferner können sie über einen weiten Bereich variiert werden, abhängig von der Art und den Eigenschaften der Zink-und Zinklegierungsbäder, denen die Substanzen zugesetzt werden.

Tabelle 1 zeigt gemäß einer bevorzugten Ausführungsform mit Nickel als Legierungsmetall den Einfluss hinsichtlich Schichtdicke und Nickel-Einbau in den erfindungsgemäßen Elektrolyten zur Abscheidung einer Zink-Nickellegierung (bei Verwendung von 4,03*10⁻⁴ mol/l des entsprechenden Additivs):

**Tabelle 1**

| Beispiel | Verbindung | Glanz | Schichtdicke [µm] j=3A/dm² | Schichtdicke [µm] j=0,5A/dm² | Ni-Konz. [%] j=3 A/dm² | Ni-Konz. [%] j=0,5 A/dm² |
|---|---|---|---|---|---|---|
| 1 | 1-Benzyl-3-carbamoyl-pyridiniumchlorid | ++ | 6,5 | 2,9 | 14,8 | 12,7 |
| 2 | 1-Benzyl-3-carboxy-pyridiniumchlorid | + | 5,8 | 2,4 | 15,6 | 13,5 |
| 3 | 1,1'-(Xylenyl)-3,3'-bis-carboxy-bispyridinium-dibromid | +++ | 7,4 | 2,5 | 15,3 | 13,4 |
| 4 | 1,1'-(Xylenyl)-3,3'-bis-carbamoyl-bispyridinium-dichlorid | +++ | 7,1 | 2,2 | 16,5 | 13,3 |
| 5 | 1-(4'-Fluor-benzyl)-3-carbamoyl-pyridinium-chlorid | +++ | 6,8 | 3,5 | 14,6 | 13,6 |
| 6 | 1-(4'-Methoxy-benzyl)-3-carbamoyl-pyridinium-chlorid | ++ | 7,2 | 2 | 14,4 | 12,0 |
| 7 | 1-(1'-Methyl-naphthyl)-3-carbamoyl-pyridinium-chlorid | ++ | 8,0 | 3,5 | 15,8 | 13 |
| 8 | 1-(4'-Carboxy-benzyl)-3-carbamoyl-pyridinium-chlorid | +++ | 6 | 2 | 14,7 | 13,0 |
| 9 | 1,1'-(But-2-enyl)-3,3'-bis-carbamoyl-bispyridinium-dichlorid | ++ | 7,7 | 2,0 | 15,0 | 14,3 |
| 10 | 1-Allyl-3-carbamoyl-pyridinium-chlorid | ++ | 7,3 | 2,0 | 14,4 | 13,7 |

Wie aus Tabelle 1 ersichtlich ist, werden bei Verwendung der Carbamoyl-Verbindungen (Beispiele 1 sowie 4 bis 10) bzw. Verbindungen der Formel II (Beispiel 3) hinsichtlich der Schichtdicke, die direkt proportional zur Stromausbeute ist, sowie hinsichtlich der Nickel-Einbauraten günstigere Werte erhalten. So werden mit den erfindungsgemäßen Glanzbildnern Stromausbeuten erhalten, die bis zu 38% über den bis dato üblichen Glanzbildnern, wie z.B. 1-Benzyl-3-carboxy-pyridinium-chlorid (Beispiel 2), welches z.B. in der Schrift DE 102 23 622 B4 erwähnt ist, liegen können.

Die erfindungsgemäßen Glanzbildner können alleine oder in Kombination miteinander eingesetzt werden. Tabelle 2 zeigt den Einfluss der erfindungsgemäßen Glanzbildner bei Einsatz eines einheitlichen Masseneinsatzes:

**Tabelle 2: Einfluss der Pyridinium-Verbindungen auf Zink-Nickel-Legierungsabscheidungen (Einsatzmenge 100mg/l):**

| Beispiel | Verbindung | Glanz | Schichtdicke [µm] j=3A/dm² | Schichtdicke [µm] j=0,5A/dm² | Ni-Konz. [%] j = 3 A/dm² | Ni-Konz. [%] j = 0,5 A/dm² |
|---|---|---|---|---|---|---|
| 1 | 1-Benzyl-3-carbamoyl-pyridiniumchlorid | ++ | 6,5 | 2,9 | 14,8 | 12,7 |
| 2 | 1-Benzyl-3-carboxy-pyridiniumchlorid | + | 5,8 | 2,4 | 15,6 | 13,5 |
| 11 | 1,1'-(Xylenyl)-3,3'-bis-carboxy-bispyridinium-dibromid | ++ | 7,4 | 2,2 | 15,0 | 12,0 |
| 12 | 1,1'-(Xylenyl)-3,3'-bis-carbamoyl-bispyridinium-dichlorid | ++ | 7,5 | 2,2 | 14,5 | 12,7 |
| 13 | 1-(4'-Methoxy-benzyl)-3-carbamoyl-pyridinium-chlorid | ++ | 8,1 | 2,7 | 15,0 | 13,5 |
| 14 | 1-(1'-Methyl-naphthyl)-3-carbamoyl-pyridinium-chlorid | ++ | 7,5 | 2,4 | 14,8 | 12,5 |
| 15 | 1-(4'-Carboxy-benzyl)-3-carbamoyl-pyridinium-chlorid | +++ | 8,3 | 2,6 | 14,7 | 12,8 |
| 16 | 1-(3-Sulfopropyl)-3-carbamoyl-pyridinium-betain (300 mg/l) | ++ | 6,2 | 2,2 | 14,0 | 12,2 |
| 17 | 1-Benzyl-3-carbamoyl-pyridinium-chlorid (20 mg/I)/ 1-(4'-Fluor-benzyl)-3-carbamoyl-pyridinium-chlorid (80 mg/l) | ++ | 7,1 | 2,7 | 14,1 | 12,8 |

Durch Variation der am Stickstoff befindlichen Gruppen können, je nach eingesetztem Aryl- oder Alkyl-Rest und den daran befindlichen Gruppen, unterschiedliche Nickel-Einbauraten erhalten werden. Ebenso können, wie aus Tabelle 1 und 2 ersichtlich, je nach eingesetztem Glanzbildner und seiner Menge unterschiedliche Effekte hinsichtlich Glanz, Schichtdicke und Nickeleinbauraten über einen großen Stoff- und Massenmengen-Bereich erhalten werden und die erfindungsgemäßen Bäder je nach gewünschter Schichtperformance durch Wahl des Additivs oder eines Additivgemisches gezielt eingestellt werden.

Ein weiterer Vorteil der erfindungsgemäßen Zink-Nickel-Legierungsbäder ist, dass in der Regel auf den Einsatz von aromatischen Aldehyden und Tellurit als Glanzbildner gänzlich verzichtet werden kann.

Neben der Verwendung in Zink-Nickel-Elektrolyten können die oben genannten quaternären Pyridinium-Verbindungen unter Verwendung polymerer bzw. oligomerer Harnstoffderivate der allgemeinen Formel VI, wie sie in der Schrift US 6,652,728 oder in Schrift US 5,435,898 beschrieben sind, auch in den erfindungsgemäßen Bädern, insbesondere in alkalischen cyanidfreien Zink, Zink-Eisen und Zink-Cobalt-Elektrolytbädern, eingesetzt werden.

Gemäß einer bevorzugten Ausführungsform betrifft die Erfindung ein wie oben beschriebenes Elektrolytbad, das weiterhin ein lösliches Polymer der allgemeinen Formel VI umfasst 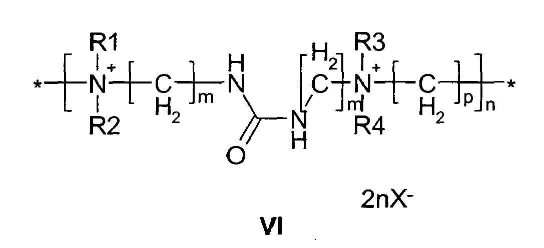 worin R¹, R², R³ und R⁴ gleich oder verschieden sein können und für einen substituierten oder unsubstituierten Kohlenwasserstoffrest mit 1 bis 6 Kohlenstoffatomen stehen, m für eine ganze Zahl von 1 bis 4 steht, n für eine ganze Zahl größer als 1 steht, p für eine ganze Zahl von 3 bis 12 steht und X- für ein Gegenion steht.

Bevorzugt sind Verbindungen der Formel VI, worin R¹, R², R³ und R⁴ für Methyl, Ethyl, Hydroxyethyl, Propyl oder Butyl stehen, m 2 oder 3 ist und X⁻ für ein Halogenid oder Pseudohalogenid steht.

Produkte der allgemeinen Formel VI sind im Handel u.a. unter den Handelsbezeichnungen Mirapol^{©} WT und AD-1 erhältlich.

Überraschend wurde gefunden, dass die erfindungsgemäß eingesetzten Pyridinium-Verbindungen hinsichtlich ihrer Glanzes und ihrer Stabilität den in US 6,652,728 beschriebenen Nicotinsäure-Analoga überlegen sind.

Dabei ist das eingesetzte polymere Harnstoff-Derivat (Formel VI) in dem erfindungsgemäßen Bad in einer Menge von 0,1 bis 50 g/l, vorzugsweise 0,25 bis 10 g/l enthalten. Die Menge des eingesetzten erfindungsgemäßen Pyridinium-Zusatzes in dem erfindungsgemäßen Bad beträgt 0,005 bis 0,5 g/l, vorzugsweise 0,01 bis 0,2 g/l.

Tabelle 3 zeigt den Vergleich zwischen einem erfindungsgemäßen Bad, das 1-Benzyl-3-carbamoyl-pyridinium-chlorid als Glanzbildner enthält und einem Bad der Schrift US 6,652,728.

**Tabelle 3: Alkalische Zink-Elektrolyten**

| Beispiel | Glanz | Schichtdicke [µm] j=3A/dm² | Schichtdicke [µm] j=0,5A/dm² |
|---|---|---|---|
| 18 | ++ | 3,12 | 2,09 |
| Bad gemäß US 6,652,728 | + | 3,06 | 1,96 |

Wie aus der Tabelle 3 ersichtlich ist, werden auch hier glänzendere Schichten bei gleichzeitig geringfügig höherer Stromausbeute erhalten.

Im Falle von Zink-Eisen-Elektrolyten liegt der Eisengehalt zwischen 0,4 bis 1%.

Die erfindungsgemäßen Bäder können neben den oben genannten Additiven bekannte Einebner wie 3-Mercapto-1,2,4-triazol und/oder Thioharnstoff enthalten, wobei Thioharnstoff oftmals bevorzugt wird.

Überraschend wurde gefunden, dass bei den oben genannten erfindungsgemäßen Elektrolyten auf die übliche Verwendung aromatischer Aldehyde bzw. deren Bisulfit-Addukte als zusätzliche Glanzbildner, wie z.B. 4-Hydroxybenzaldehyd, 4-Hydroxy-3-methoxybenzaldehyd, 3,4-Dimethoxybenzaldehyd, 3,4-Methylendioxybenzaldehyd, 2-Hydroxybenzaldehyd oder Gemischen davon verzichtet werden kann.

Ein weiterer interessanter Aspekt der erfindungsgemäßen Glanzbildner und der damit verbundenen erfindungsgemäßen Elektrolyten besteht in der unterschiedlichen Kristallit-Zusammensetzung der abgeschiedenen Schichten.

Die erfindungsgemäßen wässrigen alkalischen Bäder können im allgemeinen für alle Arten von Substraten verwendet werden, auf welchen Zink- und Zink-Legierungen abgeschieden werden können. Beispiele zweckdienlicher Substrate schließen Weichstahl, Federstahl, Chromstahl, Chrom-Molybdän-Stahl, Kupfer, Kupfer-Zink-Legierungen etc. ein.

Ein weiterer Gegenstand der Erfindung ist daher ein Verfahren zur galvanischen Abscheidung von Zink- und Zink-Legierungsüberzügen auf üblichen Substraten, das dadurch gekennzeichnet ist, dass als Bad ein Bad mit den oben genannten Zusammensetzungen verwendet wird. Vorzugsweise erfolgt bei dem erfindungsgemäßen Verfahren die Abscheidung der Überzüge bei einer Stromdichte von 0,01 A/dm² bis 10 A/dm² sowie bei einer Temperatur im Bereich von 15 bis 50°C, vorzugsweise 20-30°C, besonders bevorzugt etwa 25°C.

Das erfindungsgemäße Verfahren kann bei der Anwendung für Massenteile beispielsweise als Trommelgalvanisierverfahren und zur Abscheidung auf größeren Werkstücken als Gestellgalvanisierverfahren durchgeführt werden. Dabei werden Anoden verwendet, die löslich sein können, wie beispielsweise Zinkanoden, die gleichzeitig als Zink-Ionen-Quelle dienen, damit das auf der Kathode abgeschiedene Zink durch Auflösen von Zink an der Anode substituiert wird.

Andererseits können auch unlösliche Anoden (z.B. platinierte Titanmischoxid-Anoden) eingesetzt werden, wobei die dem Elektrolyten entzogenen Zink-Ionen und/oder den weiteren Metallionen bei Legierungsabscheidungen auf andere Weise wieder zugesetzt werden müssen, z.B. unter Verwendung eines Zinklösebehälters.

Wie bei der galvanischen Abscheidung möglich, kann auch das erfindungsgemäße Verfahren unter Lufteinblasung, mit Warenbewegung oder ohne Bewegung, betrieben werden, ohne dass sich hierfür irgendwelche Nachteile für die erhaltenen Überzüge ergeben. Zur Vermeidung bzw. Reduzierung von Oxidationen der zugesetzten Additive kann mit der Trennung der Elektrodenräume bzw. mit der Verwendung von Membrananoden gearbeitet werden.

Als Stromquelle dienen handelsübliche Gleichstromrichter oder Pulsgleichrichter.

Die nachfolgenden Beispiele erläutern die Erfindung, ohne jedoch die Erfindung auf diese zu beschränken.

### Herstellbeispiel 1: Synthese von 1-(4'-Methoxy-benzyl)-3-carbamoyl-pyridinium-chlorid

In einem 100-ml-Rundkolben mit Rückflusskühler werden 60 ml Wasser, 9,2 g Nicotinsäureamid (98%-ig) (0,0738 mol), 11,68 g 4-Methoxybenzylchlorid (99%-ig) (0,07378 mol) gegeben und 24 Stunden unter Rückfluss erhitzt. Nach beendeter Reaktion wird das Wasser im Vakuum entfernt und der Rückstand in 200 ml Ethanol aufgenommen und eine weitere Stunde unter Rückfluss erhitzt. Anschließend wird der Reaktionsansatz auf 4°C abgekühlt und der anfallende weiße Feststoff abfiltriert und im Vakuum getrocknet.

Es wurden 16,92 g eines weißen Feststoffes erhalten (82,26% d. Th.).

### Herstellbeispiel 2: Synthese von 1-(4'-Chlor-benzyl)-3-carbamoyl-pyridinium-chlorid

In einem 100-ml-Rundkolben mit Rückflusskühler werden 60 ml Ethanol, 10 g Nicotinsäureamid (98%-ig) (0,0802 mol), 13,05 g 4-Chlor-benzylchlorid (99%-ig) (0,0802 mol) gegeben und 24 Stunden unter Rückfluss erhitzt. Nach beendeter Reaktion wird der feste Rückstand für weiter 15 Minuten in 200 ml eines Ethanof/Methanol-Gemisches gekocht und anschließend auf 4°C abgekühlt. Der anfallende Feststoff wird abfiltriert und im Vakuum getrocknet.

Es wurden 18,82 g eines weißen Feststoffes erhalten (82,87 % d. Th.).

### Herstellbeispiel 3: Synthese von 1-(4'-Carboxy-benzyl)-3-carbamoyl-pyridinium-chlorid

In einem 100-ml-Rundkolben mit Rückflusskühler werden 60 ml Ethanol, 7,09 g Nicotinsäureamid (98%-ig) (0,0569 mol), 10,22 g 4-Chlor-benzoesäure (95%-ig) (0,0569 mol) gegeben und 24 Stunden unter Rückfluss erhitzt. Nach beendeter Reaktion wird der Reaktionsansatz auf 4°C abgekühlt, der anfallende Feststoff abfiltriert und im Vakuum getrocknet.

Es wurden 13,21 g eines weißen Feststoffes erhalten (79,21 % d. Th.).

### Herstellbeispiel 4: Synthese von 1-(1'-Methyl-naphthyl)-3-carbamoyl-pyridinium-chlorid

In einem 100-ml-Rundkolben mit Rückflusskühler werden 60 ml Ethanol, 10 g Nicotinsäureamid (98%-ig) (0,0802 mol), 15,75 g 1-Chlor-methyl-naphthalin (90%-ig) (0,0802 mol) gegeben und 24 Stunden unter Rückfluss erhitzt. Nach beendeter Reaktion wird der feste Rückstand für weiter 15 Minuten in 200 ml eines Ethanol/Methanol-Gemisches (75:25) gekocht und anschließend auf 4°C abgekühlt. Der anfallende Feststoff wird abfiltriert und im Vakuum getrocknet.

Es wurden 19,37 g eines weißen Feststoffes erhalten (80,84 % d. Th.).

### Herstellbeispiel 5: Synthese von 1-(4'-Fluor-benzyl)-3-carbamoyl-pyridinium-chlorid

In einem 100-ml-Rundkolben mit Rückflusskühler werden 60 ml Ethanol, 10 g Nicotinsäureamid (98%-ig) (0,0802 mol), 11,72 g 4-Fluor-benzylchlorid (99%-ig) (0,0802 mol) gegeben und 24 Stunden unter Rückfluss erhitzt. Nach beendeter Reaktion wird der feste Rückstand für weiter 15 Minuten in 200 ml eines Ethanol/Methanol-Gemisches gekocht und anschließend auf 4°C abgekühlt. Der anfallende Feststoff wird abfiltriert und im Vakuum getrocknet.

Es wurden 18,13 g eines weißen Feststoffes erhalten (84,76 % d. Th.).

### Herstellbeispiel 6: Synthese von 1,1'-(Xylenyl)-3,3'-bis-carbamoyl-bispyridinium-dichlorid

In einem 100-ml-Rundkolben mit Rückflusskühler werden 60 ml Ethanol, 10 g

Nicotinsäureamid (98%-ig) (0,0802 mol), 7,16 g α,α'-Dichlor-p-xylol (98%-ig) (0,0401 mol) gegeben und 24 Stunden unter Rückfluss erhitzt. Nach beendeter Reaktion wird der feste Rückstand für weiter 15 Minuten in 200 ml eines Ethanol/Methanol-Gemisches gekocht und anschließend auf 4°C abgekühlt. Der anfallende Feststoff wird abfiltriert und im Vakuum getrocknet.

Es wurden 12,29 g eines weißen Feststoffes erhalten (73,13 % d. Th.).

### Herstellbeispiel 7: Synthese von 1-(4'-Chlor-benzyl)-3-carbamoyl-pyridinium-chlorid

In einem 100-ml-Rundkolben mit Rückflusskühler werden 60 ml Ethanol, 10 g Nicotinsäureamid (98%-ig) (0,0802 mol), 6,26 g Allylchlorid (98%-ig) (0,0802 mol) gegeben und 24 Stunden unter Rückfluss erhitzt. Nach beendeter Reaktion wird der Reaktionsansatz auf 4°C abgekühlt und der anfallende Feststoff wird abfiltriert und im Vakuum getrocknet.

Es wurden 9,25 g eines weißen Feststoffes erhalten (58,07 % d. Th.).

### Herstellbeispiel 8: Synthese von 1-Benzyl-3-carbamoyl-pyridinium-chlorid

In einem 100-ml-Rundkolben mit Rückflusskühler werden 60 ml Ethanol, 10,83 g Chinolin (97%-ig) (0,0869 mol), 13,33 g Benzylchlorid (99%-ig) (0,10428 mol) gegeben und 24 Stunden unter Rückfluss erhitzt. Nach beendeter Reaktion wird der Reaktionsansatz auf 4°C abgekühlt, der anfallende Feststoff abfiltriert und aus einem Liter Ethanol umkristallisiert.

Es wurden 14,51 g eines weißen kristallinen Feststoffes erhalten (67,14% d. Th.).

### Herstellbeispiel 9: Synthese von 1,1'-(But-2-enyl)-3,3'-bis-carbamoyl-bispyridinium-dichlorid

In einem 100-ml-Rundkolben mit Rückflusskühler werden 60 ml Ethanol, 10 g Nicotinsäureamid (98%-ig) (0,0802 mol), 5,90 g (0,0401 mol) trans-1,4-Dichlor-2-buten (85%-ig) gegeben und 24 Stunden auf Rückfluss erhitzt Nach beendeter Reaktion wird der Reaktionsansatz auf 4°C abgekühlt und der anfallende Feststoff abfiltriert und im Vakuum getrocknet.

Es wurden 13,64 g eines weißen Feststoffes erhalten (92,19% d. Th.).

### Herstellbeispiel 10: 1,3-Bis[(2-hydroxy-ethyl)-methyl-amino]-propan-2-ol

163,24 ml (2 mol) 2-(Methylamino)-ethanol (98%-ig) werden in einem 500-ml-Dreihalskolben vorgelegt und auf 130°C erhitzt. Anschließend werden innerhalb von 40 Minuten langsam 96,44 ml 1,3-Dichlorpropanol so zugetropft, dass die Reaktionstemperatur 135°C nicht überschreitet. Nach beendeter Zugabe wird das Reaktionsgemisch noch eine Stunde auf 130°C erhitzt und langsam auf Raumtemperatur (innerhalb von 3 Stunden) auf Raumtemperatur angekühlt. Das entstandene braune Öl wird in 1 l Wasser gelöst und die resultierende Lösung mit 102,95 g Natriumcarbonat versetzt und das Reaktionsgemisch eine Stunde gerührt.

Anschließend wird das überschüssige Wasser im Vakuum entfernt und der Rückstand in 300 ml Ethanol aufgenommen, eine Stunde unter Rückfluss erhitzt und der Reaktionsansatz bei Raumtemperatur filtriert. Das überschüssige Lösemittel wird im Vakuum entfernt und im Vakuum getrocknet.

Es entstanden 224,4 g eines braunen viskosen Öls (80,37% d. Th.)

### Herstellbeispiel 11: Synthese von 1,1',1"-(Mesitylenyl)-3,3',3"-tri-carbamoyl-tri-pyridinium-tribromid

In einem 100-ml-Rundkolben mit Rückflusskühler werden 60 ml Ethanol, 10 g Nicotinsäureamid (98%-ig) (0,0802 mol), 9,54 g α,α',α"-Tribrom-mesitylen (0,02674 mol) gegeben und 24 Stunden unter Rückfluss erhitzt. Nach beendeter Reaktion wird der feste Rückstand für weiter 15 Minuten in 200 ml eines Ethanol/Methanol-Gemisches gekocht und anschließend auf 4°C abgekühlt. Der anfallende Feststoff wird abfiltriert und im Vakuum getrocknet.

Es wurden 12,02 g eines weißen Feststoffes erhalten (62 % d. Th.).

### Anwendungsbeispiel 1:

Es wird ein Elektrolyt mit folgender Zusammensetzung verwendet:

| | |
|---|---|
| 12 g/l | Zn(OH)₂ |
| 9,55 g/l | NiSO₄*6H₂O |
| 120 g/l | NaOH |
| 36 g/l | Tetraethylenpentamin |
| 100 mg/l | 1-Benzyl-3-carbamoyl-pyridinium-chlorid |

250 ml des Elektrolyten werden in eine Hullzelle gefüllt. Als Anode dient eine Nickelanode. Das Kathodenblech wird 15 min bei 1A beschichtet. Nach beendeter Beschichtung wird das Blech abgespült und unter Pressluft getrocknet. Die Schichtdickemessung erfolgt an zwei Punkten 3 cm unteren Rand und 2,5 cm vom rechten und linken Rand bei hoher (3 A/dm²) und niedriger Stromdichte (0,5 A/dm²) An eben diesen Stellen erfolgt auch die Ni-Gehaltsmessung. Gemessen wird mit XRF an vier verschiedenen Stellen der jeweiligen Position, um so Messfehler so gering wie möglich zu halten. Es wurde eine sehr glänzende Abscheidung erhalten.

Folgende Schichtdicken und Nickel-Gehalte wurden erhalten:

| Stromdichte [A/dm²] | Schichtdicke [µm] | Ni-Konz. [%] |
|---|---|---|
| 3,0 | 6,5 | 14,8 |
| 0,5 | 2,9 | 12,7 |

### Anwendungsbeispiel 2 (Vergleichsbeispiel gemäß DE 102 23 622 A1):

Anwendungsbeispiel 1 wird mit der Ausnahme wiederholt, dass ein Elektrolyt mit der folgenden Zusammensetzung verwendet wird:

| | |
|---|---|
| 12 g/l | Zn(OH)₂ |
| 9,55 g/l | NiSO₄*6H₂O |
| 120 g/l | NaOH |
| 36 g/l | Tetraethylenpentamin |
| 100 mg/l | 1-Benzyl-3-carboxy-pyridinium-chlorid |

Es wurde eine glänzende Abscheidung erhalten mit folgenden Schichtdicken und Nickel-Gehalten:

| Stromdichte [A/dm²] | Schichtdicke [µm] | Ni-Konz. [%] |
|---|---|---|
| 3,0 | 5,8 | 15,6 |
| 0,5 | 2,4 | 13,5 |

### Anwendungsbeispiel 3:

Anwendungsbeispiel 1 wird mit der Ausnahme wiederholt, dass ein Elektrolyt mit der folgenden Zusammensetzung verwendet wird:

| | |
|---|---|
| 12 g/l | Zn(OH)₂ |
| 9,55 g/l | NiSO₄*6H₂O |
| 120 g/l | NaOH |
| 36 g/l | Tetraethylenpentamin |
| 166 mg/l | 1,1'-(Xylenyl)-3,3'-bis-carboxy-bis-pyridinium-dibromid |

Es wurde eine sehr glänzende Abscheidung erhalten mit folgenden Schichtdicken und Nickel-Gehalten:

| Stromdichte [A/dm²] | Schichtdicke [µm] | Ni-Konz. [%] |
|---|---|---|
| 3,0 | 7,4 | 15,3 |
| 0,5 | 2,5 | 13,4 |

### Anwendungsbeispiel 4:

Anwendungsbeispiel 1 wird mit der Ausnahme wiederholt, dass ein Elektrolyt mit der folgenden Zusammensetzung verwendet wird:

| | |
|---|---|
| 12 g/l | Zn(OH)₂ |
| 9,55 g/l | NiSO₄*6H₂O |
| 120 g/l | NaOH |
| 36 g/l | Tetraethylenpentamin |
| 163,4 mg/l | 1,1'-(Xylenyl)-3,3'-bis-carbamoyl-bis-pyridinium-dichlorid |

Es wurde eine sehr glänzende Abscheidung erhalten mit folgenden Schichtdicken und Nickel-Gehalten:

| Stromdichte [A/dm²] | Schichtdicke [µm] | Ni-Konz. [%] |
|---|---|---|
| 3,0 | 7,1 | 16,5 |
| 0,5 | 2,2 | 13,3 |

### Anwendungsbeispiel 5:

Anwendungsbeispiel 1 wird mit der Ausnahme wiederholt, dass ein Elektrolyt mit der folgenden Zusammensetzung verwendet wird:

| | |
|---|---|
| 12 g/l | Zn(OH)₂ |
| 9,55 g/l | NiSO₄*6H₂O |
| 120 g/l | NaOH |
| 36 g/l | Tetraethylenpentamin |
| 108 mg/l | 1-(4'-Fluor-benzyl)-3-carbamoyl-pyridinium-chlorid |

Es wurde eine sehr glänzende Abscheidung erhalten mit folgenden Schichtdicken und Nickel-Gehalten:

| Stromdichte [A/dm²] | Schichtdicke [µm] | Ni-Konz. [%] |
|---|---|---|
| 3,0 | 6,8 | 14,6 |
| 0,5 | 3,5 | 13,6 |

### Anwendungsbeispiel 6:

Anwendungsbeispiel 1 wird mit der Ausnahme wiederholt, dass ein Elektrolyt mit der folgenden Zusammensetzung verwendet wird:

| | |
|---|---|
| 12 g/l | Zn(OH)₂ |
| 9,55 g/l | NiSO₄*6H₂O |
| 120 g/l | NaOH |
| 36 g/l | Tetraethylenpentamin |
| 88 mg/l | 1-(4'-Methoxy-benzyl)-3-carbamoyl-pyridinium-chlorid |

Es wurde eine sehr glänzende Abscheidung erhalten mitfolgenden Schichtdicken und Nickel-Gehalten:

| Stromdichte [A/dm²] | Schichtdicke [µm] | Ni-Konz. [%] |
|---|---|---|
| 3,0 | 7,2 | 14,4 |
| 0,5 | 2,0 | 12,0 |

### Anwendungsbeispiel 7:

Anwendungsbeispiel 1 wird mit der Ausnahme wiederholt, dass ein Elektrolyt mit der folgenden Zusammensetzung verwendet wird:

| | |
|---|---|
| 12 g/l | Zn(OH)₂ |
| 9,55 g/l | NiSO₄*6H₂O |
| 120 g/l | NaOH |
| 36 g/l | Tetraethylenpentamin |
| 123,5 mg/l | 1-(1'-Methyl-naphthyl)-3-carbamoyl-pyridinium-chlorid |

Es wurde eine sehr glänzende Abscheidung erhalten mitfolgenden Schichtdicken und Nickel-Gehalten:

| Stromdichte [A/dm²] | Schichtdicke [µm] | Ni-Konz. [%] |
|---|---|---|
| 3,0 | 8,0 | 15,8 |
| 0,5 | 3,5 | 13,0 |

### Anwendungsbeispiel 8:

Anwendungsbeispiel 1 wird mit der Ausnahme wiederholt, dass ein Elektrolyt mit der folgenden Zusammensetzung verwendet wird:

| | |
|---|---|
| 12 g/l | Zn(OH)₂ |
| 9,55 g/l | NiSO₄*6H₂O |
| 120 g/l | NaOH |
| 36 g/l | Tetraethylenpentamin |
| 120,6 mg/l | 1-(4'-Carboxy-benzyl)-3-carbamoyl-pyridinium-chlorid |

Es wurde eine sehr glänzende Abscheidung erhalten mitfolgenden Schichtdicken und Nickel-Gehalten:

| Stromdichte [A/dm²] | Schichtdicke [µm] | Ni-Konz. [%] |
|---|---|---|
| 3,0 | 6,0 | 14,7 |
| 0,5 | 2,0 | 13,0 |

### Anwendungsbeispiel 9:

Anwendungsbeispiel 1 wird mit der Ausnahme wiederholt, dass ein Elektrolyt mit der folgenden Zusammensetzung verwendet wird:

| | |
|---|---|
| 12 g/l | Zn(OH)₂ |
| 9,55g/l | NiSO₄*6H₂O |
| 120 g/l | NaOH |
| 36 g/l | Tetraethylenpentamin |
| 139,9 mg/l | 1,1'-(But-2-enyl)-3,3'-bis-carbamoyl-pyridinium-dichlorid |

Es wurde eine sehr glänzende Abscheidung erhalten mit folgenden Schichtdicken und Nickel-Gehalten:

| Stromdichte [A/dm²] | Schichtdicke [µm] | Ni-Konz. [%] |
|---|---|---|
| 3,0 | 7,7 | 15,0 |
| 0,5 | 2,0 | 14,3 |

### Anwendungsbeispiel 10:

Anwendungsbeispiel 1 wird mit der Ausnahme wiederholt, dass ein Elektrolyt mit der folgenden Zusammensetzung verwendet wird:

| | |
|---|---|
| 12 g/l | Zn(OH)₂ |
| 9,55 g/l | NiSO₄*6H₂O |
| 120 g/l | NaOH |
| 36 g/l | Tetraethylenpentamin |
| 76,85 mg/l | 1-Allyl-3-carbamoyl-pyridinium-chlorid |

Es wurde eine sehr glänzende Abscheidung erhalten mit folgenden Schichtdicken und Nickel-Gehalten:

| Stromdichte [A/dm²] | Schichtdicke [µm] | Ni-Konz. [%] |
|---|---|---|
| 3,0 | 7,3 | 14,4 |
| 0,5 | 2,0 | 13,7 |

### Anwendungsbeispiel 11:

Anwendungsbeispiel 1 wird mit der Ausnahme wiederholt, dass ein Elektrolyt mit der folgenden Zusammensetzung verwendet wird:

| | |
|---|---|
| 12 g/l | Zn(OH)₂ |
| 9,55 g/l | NiSO₄*6H₂O |
| 120 g/l | NaOH |
| 36 g/l | Tetraethylenpentamin |
| 100 mg/l | 1,1'-(Xylenyl)-3,3'-bis-carboxy-bis-pyridinium-dibromid |

Es wurde eine sehr glänzende Abscheidung erhalten mit folgenden Schichtdicken und Nickel-Gehalten:

| Stromdichte [A/dm²] | Schichtdicke [µm] | Ni-Konz. [%] |
|---|---|---|
| 3,0 | 7,4 | 15,0 |
| 0,5 | 2,2 | 12,0 |

### Anwendungsbeispiel 12:

Anwendungsbeispiel 1 wird mit der Ausnahme wiederholt, dass ein Elektrolyt mit der folgenden Zusammensetzung verwendet wird:

| | |
|---|---|
| 12 g/l | Zn(OH)₂ |
| 9,55 g/l | NiSO₄*6H₂O |
| 120 g/l | NaOH |
| 36 g/l | Tetraethylenpentamin |
| 163,4 mg/l | 1,1'-(Xylenyl)-3,3'-bis-carbamoyl-bis-pyridinium-dichlorid |

Es wurde eine sehr glänzende Abscheidung erhalten mit folgenden Schichtdicken und Nickel-Gehalten:

| Stromdichte [A/dm²] | Schichtdicke [µm] | Ni-Konz. [%] |
|---|---|---|
| 3,0 | 7,5 | 14,5 |
| 0,5 | 2,2 | 12,7 |

### Anwendungsbeispiel 13:

Anwendungsbeispiel 1 wird mit der Ausnahme wiederholt, dass ein Elektrolyt mit der folgenden Zusammensetzung verwendet wird:

| | |
|---|---|
| 12 g/l | Zn(OH)₂ |
| 9,55 g/l | NiSO₄*6H₂O |
| 120 g/l | NaOH |
| 36 g/l | Tetraethylenpentamin |
| 88 mg/l | 1-(4'-Methoxy-benzyl)-3-carbamoyl-pyridinium-chlorid |

Es wurde eine sehr glänzende Abscheidung erhalten mit folgenden Schichtdicken und Nickel-Gehalten:

| Stromdichte [A/dm²] | Schichtdicke [µm] | Ni-Konz. [%] |
|---|---|---|
| 3,0 | 8,1 | 15,0 |
| 0,5 | 2,7 | 13,5 |

### Anwendungsbeispiel 14:

Anwendungsbeispiel 1 wird mit der Ausnahme wiederholt, dass ein Elektrolyt mit der folgenden Zusammensetzung verwendet wird:

| | |
|---|---|
| 12 g/l | Zn(OH)₂ |
| 9,55 g/l | NiSO₄*6H₂O |
| 120 g/l | NaOH |
| 36 g/l | Tetraethylenpentamin |
| 100 mg/l | 1-(1'-Methyl-naphthyl)-3-carbamoyl-pyridinium-chlorid |

Es wurde eine sehr glänzende Abscheidung erhalten mit folgenden Schichtdicken und Nickel-Gehalten:

| Stromdichte [A/dm²] | Schichtdicke [µm] | Ni-Konz. [%] |
|---|---|---|
| 3,0 | 7,5 | 14,8 |
| 0,5 | 2,4 | 12,5 |

### Anwendungsbeispiel 15:

Anwendungsbeispiel 1 wird mit der Ausnahme wiederholt, dass ein Elektrolyt mit der folgenden Zusammensetzung verwendet wird:

| | |
|---|---|
| 12 g/l | Zn(OH)₂ |
| 9,55 g/l | NiSO₄*6H₂O |
| 120 g/l | NaOH |
| 36 g/l | Tetraethylenpentamin |
| 100 mg/l | 1-(4'-Carboxy-benzyl)-3-carbamoyl-pyridinium-chlorid |

Es wurde eine sehr glänzende Abscheidung erhalten mit folgenden Schichtdicken und Nickel-Gehalten:

| Stromdichte [A/dm²] | Schichtdicke [µm] | Ni-Konz. [%] |
|---|---|---|
| 3,0 | 8,3 | 14,7 |
| 0,5 | 2,6 | 12,8 |

### Anwendungsbeispiel 16:

Anwendungsbeispiel 1 wird mit der Ausnahme wiederholt, dass ein Elektrolyt mit der folgenden Zusammensetzung verwendet wird:

| | |
|---|---|
| 12 g/l | Zn(OH)₂ |
| 9,55 g/l | NiSO₄*6H₂O |
| 120 g/l | NaOH |
| 36 g/l | Tetraethylenpentamin |
| 300 mg/l | 1-(3'-Sulfo-propyl)-3-carbamoyl-pyridinium-Betain |

Es wurde eine sehr glänzende Abscheidung erhalten mit folgenden Schichtdicken und Nickel-Gehalten:

| Stromdichte [A/dm²] | Schichtdicke [µm] | Ni-Konz. [%] |
|---|---|---|
| 3,0 | 6,2 | 14,0 |
| 0,5 | 2,2 | 12,2 |

### Anwendungsbeispiel 17:

Anwendungsbeispiel 1 wird mit der Ausnahme wiederholt, dass ein Elektrolyt mit der folgenden Zusammensetzung verwendet wird:

| | |
|---|---|
| 12 g/l | Zn(OH)₂ |
| 9,55 g/l | NiSO₄*6H₂O |
| 120 g/l | NaOH |
| 36 g/l | Tetraethylenpentamin |
| 20 mg/l | 1-Benzyl-3-carbamoyl-pyridinium-chlorid |
| 80 mg/l | 1-(4'-Fluor-benzyl)-3-carbamoyl-pyridinium-chlorid |

Es wurde eine sehr glänzende Abscheidung erhalten mit folgenden Schichtdicken und Nickel-Gehalten:

| Stromdichte [A/dm²] | Schichtdicke [µm] | Ni-Konz. [%] |
|---|---|---|
| 3,0 | 7,1 | 14,1 |
| 0,5 | 2,7 | 12,8 |

### Anwendungsbeispiel 18:

Anwendungsbeispiel 1 wird mit der Ausnahme wiederholt, dass eine Stahlanode und ein Elektrolyt mit der folgenden Zusammensetzung verwendet wird:

| | |
|---|---|
| 12,5 g/l | Zn(OH)₂ |
| 120 g/l | NaOH |
| 100 mg/l | 1-Benzyl-3-carbamoyl-pyridinium-chlorid |
| 1 g/l | eines polymeren Zusatzes gemäß Herstellbeispiel 2.2 der Schrift US 6,652,728 (ber. 100%) |

Es wurde eine sehr glänzende Abscheidung erhalten.

### Anwendungsbeispiel 19:

Es wurde ein Elektrolyt hergestellt, der zum galvanischen Abscheiden einer Zink-Eisen-Schicht geeignet ist. Der Elektrolyt hatte folgende Zusammensetzung:

| | |
|---|---|
| 12,5 g/l | ZnO |
| 120 g/l | NaOH |
| 50 mg/l | Eisen (in Form von FeSO₄*7H₂O) |
| 25 g/l | Natriumgluconat |
| 1 g/l | eines polymeren Zusatzes gemäß Herstellbeispiel 2.2 der Schrift US 6,652,728 (ber. 100%) |
| 100 mg/l | 1-Benzyl-3-carbamoyl-pyridinium-chlorid |

Ein Hullzellenblech wurde bei 1 Ampere 10 Minuten lang beschichtet. Es wird eine sehr glänzende Abscheidung erhalten. Das Hullzellenblech wurde gespült und in einer handelsüblichen Schwarzchromatierung für Zink-Eisenschichten (Tridur^{©} Schwarz Liquid ZnFe, Atotech) chromatiert. Das chromatierte Blech besaß eine gute Schwarzfärbung. Die Schichtdickeverteilung wurde nach dem oben beschriebenen Test gemessen.

### Anwendungsbeispiel 20:

Anwendungsbeispiel 1 wird mit der Ausnahme wiederholt, dass ein Elektrolyt mit der folgenden Zusammensetzung verwendet wird:

| | |
|---|---|
| 12 g/l | Zn(OH)₂ |
| 9,55 g/l | NiSO₄*6H₂O |
| 120 g/l | NaOH |
| 36 g/l | Tetraethylenpentamin |
| 4 g/l | 1,3-Bis-[(2-hydroxy-ethyl)-methyl-amino]-propan-2-ol |
| 100 mg/l | 1-Benzyl-3-carbamoyl-pyridinium-chlorid |

Es wurde eine sehr glänzende Abscheidung erhalten mit folgenden Schichtdicken und Nickel-Gehalten:

| Stromdichte [A/dm²] | Schichtdicke [µm] | Ni-Konz. [%] |
|---|---|---|
| 3,0 | 5,5 | 16,3 |
| 0,5 | 2,4 | 13,5 |

## Patentansprüche

1. Wässriges alkalisches, cyanidfreies Elektrolytbad zur Abscheidung von Zink- und Zinklegierungsschichten auf Substratoberflächen, umfassend die folgenden Komponenten:
a) eine Zink-Ionen-Quelle und gegebenenfalls eine Quelle für weitere Metallionen,
b) Hydroxidionen und
c) mindestens eine Pyridiniumverbindung der allgemeinen Formeln I bis III: 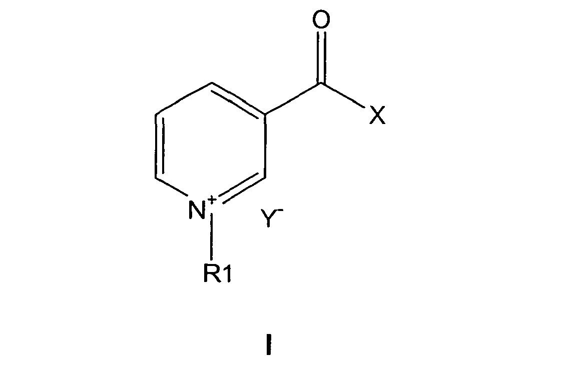 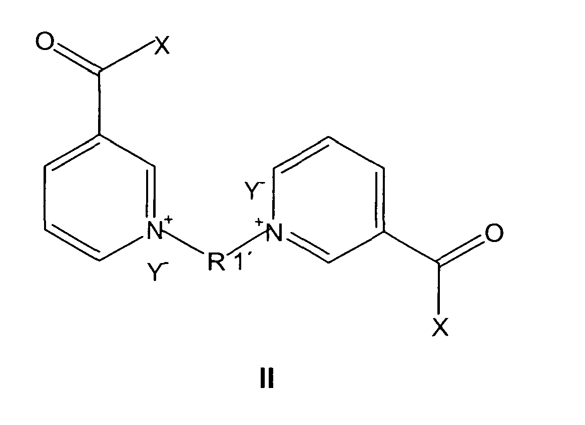 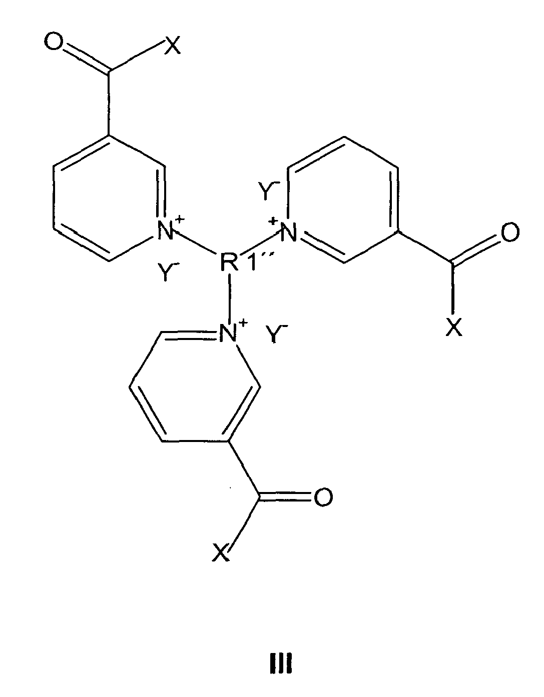 worin R¹ für einen substituierten oder unsubstituierten, gesättigten oder ungesättigten, aliphatischen oder araliphatischen Kohlenwasserstoffrest mit 1 bis 12 Kohlenstoffatomen steht,
R¹' für einen zweiwertigen substituierten oder unsubstituierten, gesättigten oder ungesättigten, aliphatischen oder araliphatischen Kohlenwasserstoffrest mit 1 bis 12 Kohlenstoffatomen steht,
R¹" für einen dreiwertigen substituierten oder unsubstituierten, gesättigten oder ungesättigten, aliphatischen oder araliphatischen Kohlenwasserstoffrest mit 1 bis 12 Kohlenstoffatomen steht,
X für Hydroxyl oder NRₓR_{y} steht, worin Rₓ und R_{y} gleich oder verschieden sein können und für Wasserstoff oder lineare und/oder verzweigte Alkylgruppen mit 1 bis 12 Kohlenstoffatomen stehen und Y- ein Gegenion ist.

2. Elektrolytbad nach Anspruch 1, worin R¹ für substituiertes Aryl der nachfolgenden Formeln R1 a bis R1l steht: 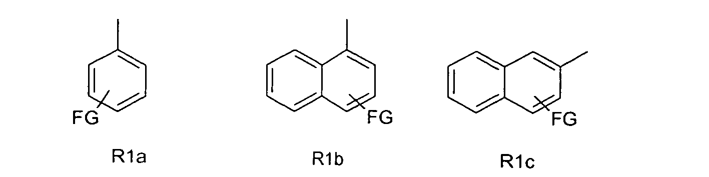 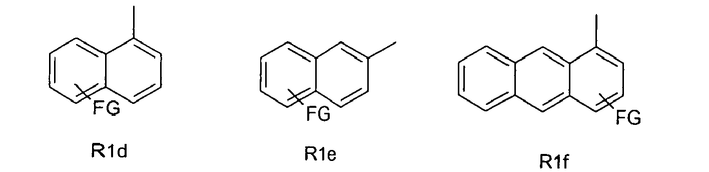 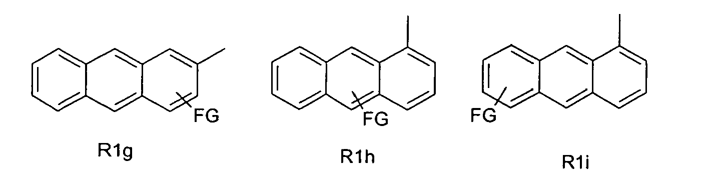 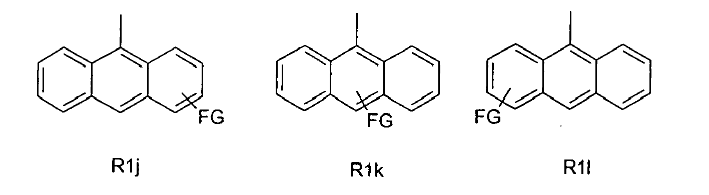 worin FG für einen Rest, ausgewählt aus der Gruppe, bestehend aus Carboxy-, Ester-, Sulfonsäure-, Carbamoyl-, Amino-, Cyano-, Alkyl-, Alkoxy-, Hydroxy-, Trifluormethyl-, Allyl-, Propargyl-, 4-Sulfobutyl-, 3-Sulfopropyl-, 4-Carboxybutyl-, 3-Carboxypropylresten, Wasserstoff und Halogenen, ausgewählt aus Fluor, Chlor und Brom, steht,
R1' für But-2-enyl, But-2-inyl oder für Aryl der nachfolgenden Formeln R1'a bis r steht: 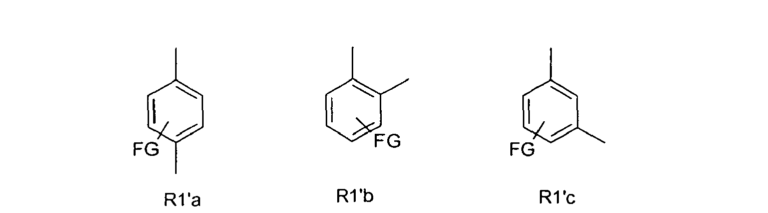 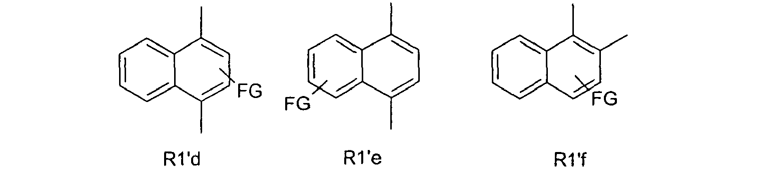 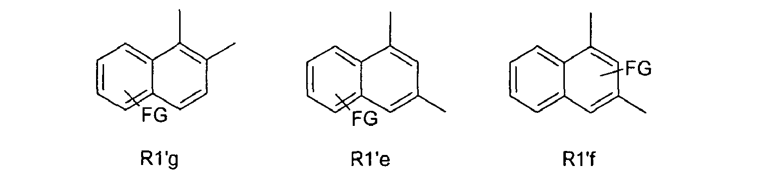 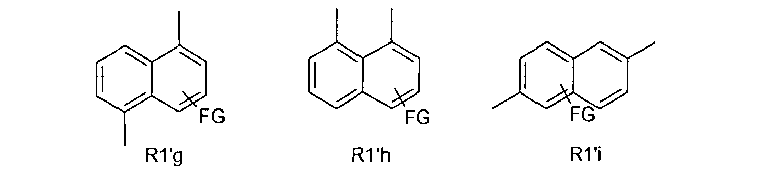 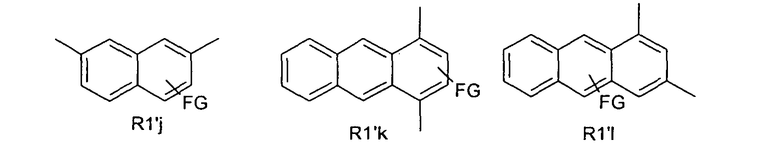 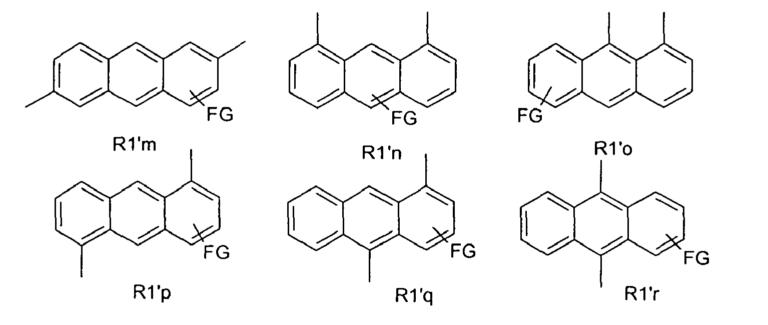 worin FG für einen Rest, ausgewählt aus der Gruppe, bestehend aus Carboxy-, Ester-, Sulfonsäure-, Carbamoyl-, Amino-, Cyano-, Alkyl-, Alkoxy-, Trifluormethylresten, Wasserstoff und Halogenen, ausgewählt aus Fluor, Chlor und Brom, steht, wobei alle Ringe oder einzelne anellierte Ringe substituiert sein können,
R¹'' für 1,3,5-Mesityl, 1,2,4-Mesityl oder 1,2,3-Mesityl steht,
X für NH₂ oder OH steht und
Y ein Halogenid oder Pseudohalogenid ist.

3. Elektrolytbad für Zink-Nickel-Legierungsabscheidungen nach den Ansprüchen 1 und 2, weiterhin umfassend mindestens ein Komplexierungsreagenz der allgemeinen Formel IV oder V: 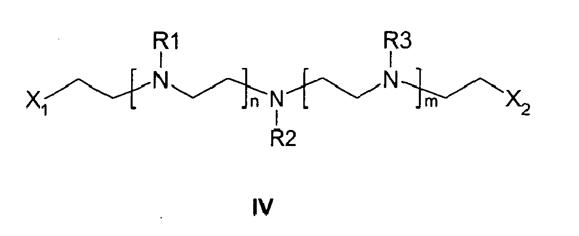 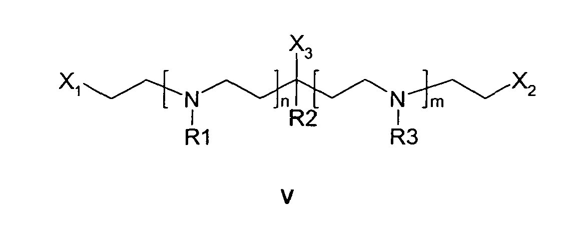 worin X₁, X₂ und X₃ gleich oder verschieden sein können und für eine Hydroxyl-, Alkoxy-, primäre, sekundäre oder tertiäre Aminogruppe stehen,
R¹, R² und R³ gleich oder verschieden sein können und für eine C₁-C₁₂-Alkoxyl-, -Allyl- oder -Propargylgruppe stehen und
n und m gleich oder verschieden sein können und für eine ganze Zahl von 0 bis 5 stehen.

4. Elektrolytbad nach den Ansprüchen 1 und 2, weiterhin umfassend ein lösliches Polymer der allgemeinen Formel VI 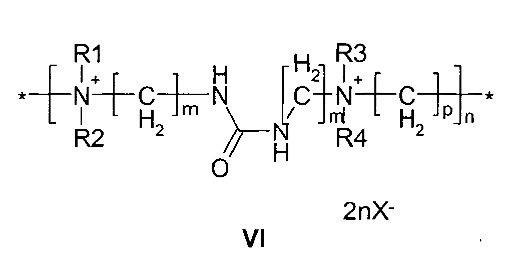 worin R¹, R², R³ und R⁴ gleich oder verschieden sein können und für einen substituierten oder unsubstituierten Kohlenwasserstoffrest mit 1 bis 6 Kohlenstoffatomen stehen,
m für eine ganze Zahl von 1 bis 4 steht,
n für eine ganze Zahl größer als 1 steht,
p für eine ganze Zahl von 3 bis 12 steht und
X- für ein Gegenion steht.

5. Elektrolytbad nach Anspruch 4, worin R¹, R², R³ und R⁴ für Methyl, Ethyl, Hydroxyethyl, Propyl oder Butyl stehen,
m 2 oder 3 ist und
X⁻ für ein Halogenid oder Pseudohalogenid steht.

6. Bad nach den Ansprüchen 1 bis 5, enthaltend eine Kombination der Pyridiniumverbindungen der Formeln I bis III.

7. Bad nach den Ansprüchen 1 bis 6, enthaltend eine Kombination der Komplexierungsreagenzien IV, V und/oder VI.

8. Elektrolytbad nach den Ansprüchen 3 und 4, umfassend
1 bis 100 g/l Zinkionen,
0,1 bis 50 g/l Legierungsmetallionen,
5 bis 100 g/l mindestens einer Verbindung der allgemeinen Formeln IV, V und/oder VI und
0,001 bis 20 g/l mindestens einer der Verbindungen der allgemeinen Formeln I bis III oder einer Kombination davon.

9. Elektrolytbad nach Anspruch 8, enthaltend:
4 bis 30 g/l Zinkionen,
Legierungsmetallionen, ausgewählt aus Nickel-, Eisen-, Cobalt-, Managanionen und
0,01 bis 10g/l mindestens einer der Verbindungen der allgemeinen Formeln I bis III oder einer Kombination davon.

10. Elektrolytbad nach Anspruch 1, enthaltend als Legierungsmetall Nickel in einer Menge von 0,1 bis 50 g/l, Eisen in einer Menge von 10 bis 120 mg/l, Mangan in einer Menge von 10 bis 100 g/l und/oder Kobalt in einer Menge von 10 bis 120 mg/l.

11. Elektrolytbad nach Anspruch 1, enthaltend als Base ein Alkalimetallhydroxid.

12. Elektrolytbad nach Anspruch 11, worin das Alkalimetallhydroxid Natriumhydroxid und/oder Kaliumhydroxid ist und in einer Menge von 50 bis 250 g/l vorliegt.

13. Elektrolytbad nach den Ansprüchen 1 bis 12 mit einem pH-Wert von mindestens 10.

14. Verfahren zur galvanischen Abscheidung von glänzenden und ebenen Zink- und Zinklegierungsüberzügen, umfassend die Stufe des Einbringens eines zu beschichtenden Substrats in ein Bad nach den Ansprüchen 1 bis 13.

15. Verfahren nach Anspruch 14, worin das Bad bei einer Stromdichte von 0,01 bis 10 A/dm² betrieben wird.

16. Verfahren nach Anspruch 14, worin das Bad bei einer Temperatur von 15 bis 50°C betrieben wird.

17. Verfahren nach Anspruch 16, worin das Bad bei einer Temperatur von 30°C betrieben wird.

18. Verfahren nach den Ansprüchen 14 bis 17, worin die Überzüge auf einem leitenden Substrat unter Anwendung eines Trommelgalvanisierungsverfahrens abgeschieden werden.

19. Verfahren nach den Ansprüchen 14 bis 17, worin die Überzüge auf einem leitenden Substrat unter Anwendung eines Gestellgalvanisierverfahrens abgeschieden werden.

20. Verfahren nach den Ansprüchen 14 bis 19, worin ein Zinküberzug auf dem Substrat abgeschieden wird.

21. Verfahren nach den Ansprüchen 14 bis 19, worin ein Zinklegierungsüberzug auf dem Substrat abgeschieden wird.

22. Verfahren nach Anspruch 21, worin ein Überzug aus einer Zinklegierung mit einem oder mehreren Metallen aus der Gruppe, bestehend aus Kobalt, Nickel, Mangan und Eisen, auf dem Substrat abgeschieden wird.

23. Verbindungen der allgemeinen Formel III 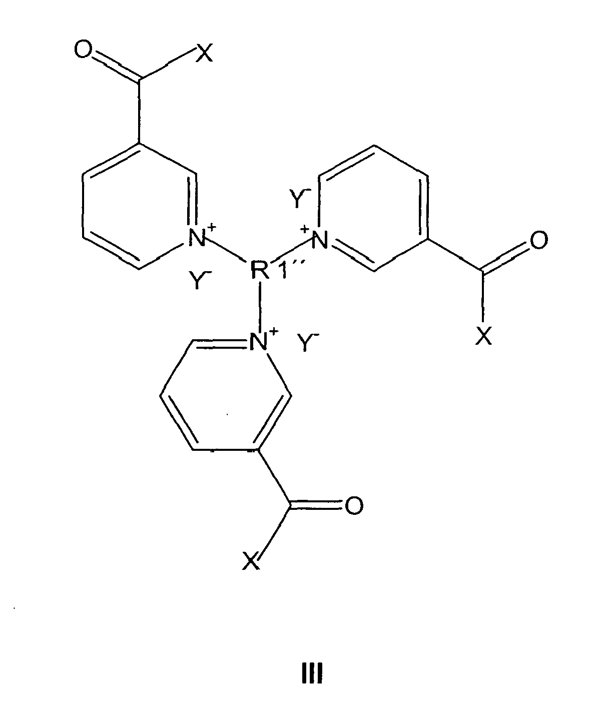 worin X, Y, R¹' und R¹" wie in Anspruch 1 definiert sind.

24. Verwendung der Verbindungen nach Anspruch 23 als Glanzbildner.
